# EUROPÄISCHE PATENTSCHRIFT

(11) **EP 3 844 515 B1**
(45) Veröffentlichungstag und Bekanntmachung des Hinweises auf die Patenterteilung: **09.07.2025**
(21) Anmeldenummer: 19758668.8
(22) Anmeldetag: 20.08.2019
(51) Int. Cl.: G01R 33/465, A61B 5/055, A61B 5/15, A61B 10/00, A61B 5/157

(54) **HANDGEHALTENES MESSGERÄT ZUR ANALYSE VON KÖRPERFLÜSSIGKEITEN MIT KERNSPINRESONANZ-SENSOREINHEIT, EINER EINHEIT ZUR AUFNAHME EINER KÖRPERFLÜSSIGKEIT, UND EINER PUNKTIONSEINHEIT ZUR MINIMAL-INVASIVEN PUNKTION EINES KÖRPERS**
HAND-HELD MEASURING DEVICE FOR ANALYZING BODY FLUIDS USING A NUCLEAR MAGNETIC RESONANCE SENSOR UNIT, A UNIT FOR RECEIVING A BODY FLUID, AND A FUNCTIONAL UNIT FOR A MINIMALLY INVASIVE PUNCTURE OF A BODY
APPAREIL DE MESURE TENU EN MAIN SERVANT À L'ANALYSE DE FLUIDES CORPORELS AVEC UNE UNITÉ DE CAPTEUR À RÉSONANCE MAGNÉTIQUE, UNE UNITÉ DE RÉCEPTION D'UN FLUIDE CORPOREL, ET UNE UNITÉ FONCTIONNELLE DE PONCTION LA MOINS INVASIVE POSSIBLE D'UN CORPS

(30) Priorität: 27.08.2018 DE 102018214459
(43) Veröffentlichungstag der Anmeldung: 07.07.2021
(73) Patentinhaber: Robert Bosch GmbH, 70442 Stuttgart (DE)
(72) Erfinder: TACER, Seda, 97816 Lohr am Main (DE); DAHL, Irene, 70619 Stuttgart (DE); STUMBER, Michael, 70825 Korntal-Muenchingen (DE); STEIN, Ralf, 73230 Kirchheim/Teck (DE); GUPTA, Sanghmitra, 70567 Stuttgart (DE); RITTLER, Stephan, 73660 Urbach (DE); COLLINS, Tom, Worcester, Worcestershire WR3 8XA (GB); HALIL, Alan, Forest Lake, Queensland 4078 (AU)
(86) Internationale Anmeldenummer: PCT/EP2019/072224
(87) Internationale Veröffentlichungsnummer: WO 2020/043547

(56) Entgegenhaltungen:
- DE-A1- 102015 111 712
- US-A1- 2003 114 785
- US-A1- 2015 018 638
- PENG WENG KUNG ET AL: "Development of miniaturized, portable magnetic resonance relaxometry system for point-of-care medical diagnosis", REVIEW OF SCIENTIFIC INSTRUMENTS, AIP, MELVILLE, NY, US, vol. 83, no. 9, 1 September 2012 (2012-09-01), pages 95115 - 95115, XP012162672, ISSN: 0034-6748, [retrieved on 20120926], DOI: 10.1063/1.4754296
- CISTOLA DAVID P ET AL: "Compact NMR relaxometry of human blood and blood components", TRAC TRENDS IN ANALYTICAL CHEMISTRY, ELSEVIER, AMSTERDAM, NL, vol. 83, 4 May 2016 (2016-05-04), pages 53 - 64, XP029727418, ISSN: 0165-9936, DOI: 10.1016/J.TRAC.2016.04.020

## Beschreibung

### Stand der Technik

In US2005/0021019 A1 ist bereits ein handgehaltenes Messgerät zur Analyse von Körperflüssigkeiten, insbesondere Blut, mit zumindest einer Kernspinresonanz-Sensoreinheit, mit zumindest einer Auswerteeinheit zur Auswertung eines von der Kernspinresonanz-Sensoreinheit gelieferten Messsignals und mit zumindest einer Aufnahmeeinheit, insbesondere Mikrokapillareinheit, zur Aufnahme der Körperflüssigkeit vorgeschlagen worden. Darüber hinaus weist das in US2005/0021019 A1 vorgeschlagene handgehaltene Messgerät eine Biopsienadel auf, welche dazu vorgesehen ist, eine Sensoreinheit in einen Körper hinein zu führen und eine Gewebeprobe aktiv aus dem Körper herauszuschneiden.

Peng Weng Kung et al. publizierte in REVIEW OF SCIENTIFIC INSTRUMENTS, AIP, Bd. 83, Nr. 9, 1. September 2012 (2012-09-01), Seiten 95115 - 95115, XP012162672, ISSN: 0034-6748, D01: 10.1063/1.4754296, eine wissenschaftliche Abhandlung mit dem Titel "Development of miniaturized, portable magnetic resonance relaxometry system for point-of-care medical diagnosis". Dabei offenbart Peng Weng Kung et al. ein Messgerät zur Analyse von Körperflüssigkeiten.

US 2015/018638 A1 beschreibt Techniken für eine nicht-invasive Messung von blutbezogenen Parametern, wobei die Messungen auf einer NMR Relaxations Technik basieren.

In der DE 10 2015 111712 A1 wird ein Teststreifen offenbart, der einen Teststreifenkörper mit einem Flüssigkeitsreservoir aufweist.

Aus der US 2003/114785 A1 ist eine Blutanalysevorrichtung bekannt, die eine Mikrokapillare umfasst, wobei die Mikrokapillare auf einem Substrat mittels einem Mikroherstellungsverfahren aufgetragen ist.

Cistola David P et al. gibt mit "Compact NMR relaxometry of human blood and blood components" in TRAC TRENDS IN ANALYTICAL CHEMISTRY, ELSEVIER, Bd. 83, 4. Mai 2016 (2016-05-04), Seiten 53 - 64, XP029727418, ISSN: 0165-9936, DOI: 10.1016/J.TRAC.2016.04.020, eine Übersichtsveröffentlichung zu kompakten NMR Messgeräten für die Blutanalyse an.

### Offenbarung der Erfindung

Die Erfindung geht aus von einem handgehaltenen Messgerät ausgebildet zur Analyse von Körperflüssigkeiten, insbesondere von Blut, mit zumindest einer Kernspinresonanz-Sensoreinheit, mit zumindest einer Auswerteeinheit ausgebildet zur Auswertung eines von der Kernspinresonanz-Sensoreinheit gelieferten Messsignals und mit zumindest einer Aufnahmeeinheit, insbesondere Mikrokapillareinheit, ausgebildet zur Aufnahme der Körperflüssigkeit.

Es wird vorgeschlagen, dass das handgehaltene Messgerät zumindest eine Punktionseinheit zur minimal-invasiven Punktion eines Körpers, insbesondere eines Kapillarbetts eines Körpers, aufweist. Vorzugsweise ist das handgehaltene Messgerät dazu vorgesehen Blut zu analysieren. Es ist auch vorstellbar, dass andere Körperflüssigkeiten, insbesondere zusätzlich zu Blut, mit dem handgehaltenen Messgerät analysierbar sind, beispielsweise Lymphe, Urin und/oder Speichel. Bevorzugt wird die Körperflüssigkeit zu einer Analyse aus dem Körper entnommen.

Bevorzugt wird zur Entnahme der Köperflüssigkeit der Körper punktiert. Bevorzugt wird bei einer Punktion ein Zugangskanal in den Körper hinein erstellt, um einen Zugang zu der sich in dem Körper befindlichen Körperflüssigkeit zu ermöglichen. Vorzugsweise wird bei einer Punktion ein Zugangskanal erstellt, durch welche die Körperflüssigkeit, insbesondere durch die Einwirkung einer durch das Messgerät hervorgerufenen Kraft, aus dem Körper austritt. Es ist insbesondere denkbar, dass die Punktionseinheit zumindest teilweise einstückig mit der Aufnahmeeinheit ausgebildet ist.

Insbesondere ist die Punktionseinheit dazu vorgesehen einen Zugangskanal zu einer Körperflüssigkeit in einen Körper hinein herzustellen. Beispielsweise umfasst die Punktionseinheit zumindest eine Stech-, Schneid-, Fräs-, Ätz- und/oder Bohreinheit zur Herstellung eines Zugangskanals in einem Körper. Vorzugsweise umfasst die Punktionseinheit eine Steuereinheit zu einer Steuerung der Punktion. Vorzugsweise umfasst die Punktionseinheit eine Sensoreinheit zu einer Erfassung eines Punktionsparameters, beispielsweise eine Eindringtiefe des hergestellten Zugangskanals in den Körper und/oder eine Härte des Körpers. Bevorzugt ist die Sensoreinheit, insbesondere zusammen mit der Steuereinheit, zu einer Regelung der Punktion vorgesehen. Vorzugsweise weist das handgehaltene Messgerät eine Anlegefläche auf, die dazu vorgesehen ist an einen Körper angelegt zu werden. Vorzugsweise liegt die Anlegefläche in einer Anlegeebene. Vorzugsweise ist die Punktionseinheit dazu vorgesehen, eine Punktion im Wesentlichen senkreckt zur Anlegeebene durchzuführen. Insbesondere ist eine Punktionsrichtung zur Erstellung eines Zugangskanals im Wesentlichen senkrecht zur Anlegeebene. Der Ausdruck "im Wesentlichen senkrecht" soll hier insbesondere eine Ausrichtung einer Richtung relativ zu einer Bezugsrichtung definieren, wobei die Richtung und die Bezugsrichtung, insbesondere in einer Ebene betrachtet, einen Winkel von 90° einschließen und der Winkel eine maximale Abweichung von insbesondere kleiner als 8°, vorteilhaft kleiner als 5° und besonders vorteilhaft kleiner als 2° aufweist. Insbesondere ist eine durch die Punktionseinheit durchgeführte Punktion als "minimal invasiv" anzusehen, wenn eine maximale Wirkfläche der Punktionseinheit zur Herstellung eines Zugangskanals in einer Ebene senkrecht zur Punktionsrichtung, insbesondere in der Anlegeebene, weniger als 5 mm², bevorzugt weniger als 2,5 mm² und besonders bevorzugt von weniger 0,5 mm² beträgt.

Vorzugsweise wird die nach der Punktion aus dem Zugangskanal austretende Körperflüssigkeit von der Aufnahmeeinheit in einem Aufnahmeraum der Aufnahmeeinheit aufgenommen. Vorzugsweise übt die Aufnahmeeinheit eine für einen Austritt aus dem Körper unterstützende Kraft auf die Körperflüssigkeit aus. Bevorzugt ist die Aufnahmeeinheit als Mikrokapillareinheit ausgebildet. Vorzugsweise weist die Aufnahmeeinheit zumindest eine Kapillare, insbesondere eine Mikrokapillare auf. Bevorzugt weist die Kapillare in einer zu einer maximalen Erstreckung einer inneren Mantelfläche der Kapillare senkrechten Ebene einen größten Innendurchmesser von weniger als 0,5 mm, besonders bevorzugt weniger als 0,3 mm auf. Bevorzugt weist die Kapillare in einer zu einer maximalen Erstreckung einer inneren Mantelfläche der Kapillare senkrechten Ebene einen größten Innendurchmesser zwischen 0,05 und 0,5 mm, besonders bevorzugt zwischen 0,1 und 0,3 mm, auf. Vorzugsweise ist ein Innenraum einer Kapillare als Aufnahmeraum zur Aufnahme der Körperflüssigkeit vorgesehen. Vorzugsweise weist der Aufnahmeraum eine Aufnahmeöffnung auf. Bevorzugt gelangt die Körperflüssigkeit durch die Aufnahmeöffnung, insbesondere auf Grund einer Kapillarkraft, in den Aufnahmeraum. Vorzugsweise liegt ein die Aufnahmeöffnung begrenzender Rand der Aufnahmeeinheit in einer Öffnungsebene, die im Wesentlichen parallel zur Anlegeebene verläuft. Vorzugsweise ist der Rand der Anlegeebene zugewandt. Vorzugsweise ist die Öffnungsebene in zumindest einem Betriebszustand des handgehaltenen Messgeräts im Wesentlichen bündig mit der Anlegeebene. Unter "zumindest im Wesentlichen bündig" soll in diesem Zusammenhang insbesondere verstanden werden, dass die Öffnungsebene einen maximalen Abstand von der Anlegeebene aufweist, der zumindest kleiner als 5 mm, bevorzugt kleiner als 1 mm, besondere bevorzugt kleiner als 0,5 mm ist.

Vorzugsweise umfasst die Kernspinresonanz-Sensoreinheit, zumindest eine Magnetfeldeinheit zur Erzeugung eines statischen Magnetfeldes. Vorzugsweise ist die Magnetfeldeinheit als Permanentmagnet ausgebildet. Es ist jedoch auch denkbar, dass die Magnetfeldeinheit als Elektromagnet ausgebildet ist, insbesondere zu einer Anpassung und/oder einem Scan einer Amplitude eines erzeugten Magnetfeldes. Bevorzugt umfasst die Kernspinresonanz-Sensoreinheit zumindest eine Sendeeinheit zu einem Aussenden und/oder Empfangen eines magnetischen Wechselfeldes. Vorzugsweise weist die Sendeeinheiten eine Induktionsspule zu einem Aussenden und/oder Empfangen eines magnetischen Wechselfeldes auf. Bevorzugt weist die Kernspinresonanz-Sensoreinheit eine Steuereinheit zu einer Durchführung eines spektroskopischen Verfahrens auf. Vorzugsweise ist die Kernspinresonanz-Sensoreinheit dazu vorgesehen, Messsignale bezüglich einer sich in dem Aufnahmeraum der Aufnahmeeinheit befindlichen Körperflüssigkeit zu erfassen.

Vorzugsweise werden von der Kernspinresonanz-Sensoreinheit erstellte Messsignale mit der Auswerteeinheit ausgewertet, insbesondere zu einer Erstellung eines Kernspinresonanz-Spektrums. Insbesondere ist die Auswerteeinheit dazu vorgesehen aus den Messsignalen der Kernspinresonanz-Sensoreinheit eine Kenngröße der Körperflüssigkeit zu ermitteln. Insbesondere ist eine Kenngröße als eine absolute und/oder relative Menge, insbesondere eine Konzentration, eines in der Körperflüssigkeit enthaltenen Stoffes ausgebildet, beispielsweise Glukose, Alkohol, Hämoglobin und/oder Laktat. Vorzugsweise ist die Auswerteeinheit in einem Gehäuse des handgehalten Messgeräts angeordnet. Es ist alternativ auch denkbar, dass die Auswerteeinheit getrennt vom handgehaltenen Messgerät ausgebildet ist, wobei die Messsignale der Kernspinresonanz-Sensoreinheit über eine, insbesondere drahtlose, Datenschnittstelle übermittelt werden. Alternativ oder zusätzlich übermittelt die Auswerteeinheit mittels der, insbesondere drahtlosen, Datenschnittstelle ein Ergebnis der Auswertung zu einer Ausgabe und/oder Speicherung an ein externes Gerät, wie beispielsweise an einen PC, an ein Smartphone, an ein Tablet, an ein Laptop o. dgl.

Unter "handgehalten" soll insbesondere verstanden werden, dass das Messgerät ohne Zuhilfenahme einer Transportmaschine und/oder einer Haltevorrichtung mit den Händen, insbesondere mit einer Hand, transportiert und insbesondere zu einer Aufnahme von Körperflüssigkeit bedienbar ist. Insbesondere beträgt die Masse des Messgeräts weniger als 10 kg, bevorzugt weniger als 5 kg und besonders bevorzugt weniger als 1 kg.

Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung des handgehaltenen Messgeräts kann eine vorteilhaft komfortable Entnahme einer Körperflüssigkeit erreicht werden. Insbesondere kann durch eine minimal invasive Punktion ein Schmerzempfinden gering gehalten werden. Insbesondere kann durch eine minimal-invasive Punktion, die aus dem Zugangskanal austretende Flüssigkeit auf ein Minimum einer zur Analyse notwendigen Menge beschränkt werden.

Des Weiteren wird vorgeschlagen, dass die Punktionseinheit zumindest ein Punktionselement, insbesondere eine Lanzette, aufweist, welches zumindest in einem zur Punktion vorgesehenen Bereich einen maximalen Außendurchmesser von weniger als 1 mm aufweist. Unter einem "Punktionselement" soll insbesondere ein Element verstanden werden, das während einer Punktion zur Erstellung eines Zugangskanals in den Körper eindringt, insbesondere eine Oberfläche des Körpers durchdringt. Vorzugsweise handelt es sich bei dem Punktionselement um ein Stechwerkzeug, insbesondere um eine Lanzette. Es ist aber auch denkbar, dass das Punktionselement als Schneidelement, Fräselement, Ätzelement, Bohrelement und/oder als ein anderes dem Fachmann als sinnvoll erscheinendes Werkzeug zur Erstellung eines Zugangskanals ausgebildet ist. Vorzugsweise weist das Punktionselement einen sich verjüngenden Endbereich auf. Vorzugsweise ist der sich verjüngende Endbereich dazu vorgesehen, während einer Punktion in den Körper einzudringen. Insbesondere weist der zur Punktion vorgesehene Bereich in einer zur Punktionsrichtung senkrechten Ebene einen maximalen Außendurchmesser von weniger als 1 mm, bevorzugt von weniger als 0,7 mm, besonders bevorzugt von weniger als 0,4 mm, auf. Es ist auch denkbar, dass das Punktionselement einteilig mit der Aufnahmeeinheit, insbesondere mit einer Kapillare, ausgebildet ist und insbesondere an einem die Aufnahmeöffnung begrenzenden Rand der Aufnahmeeinheit angeordnet ist. Durch die erfindungsgemäße Ausgestaltung des handgehaltenen Messgeräts kann vorteilhaft einfach eine minimal-invasive Punktion eines Körpers erreicht werden.

Ferner wird vorgeschlagen, dass die Punktionseinheit zur Punktion des Körpers eine, insbesondere ultraschallgetriebene, Mechanikeinheit aufweist. Unter einer "Mechanikeinheit" soll insbesondere eine Einheit zu einer Erzeugung einer Bewegung und/oder einer mechanischen Schwingung, insbesondere zu einer Punktion, verstanden werden. Vorzugsweise erzeugt die Mechanikeinheit eine Relativbewegung eines Elements der Punktionseinheit, insbesondere des Punktionselements, gegen die Anlegeebene, insbesondere entlang der Punktionsrichtung. Vorzugsweise ist die Relativbewegung zu einer Punktion des Körpers mittels des Elements, insbesondere des Punktionselements, vorgesehen. Vorzugsweise umfasst die Mechanikeinheit ein Führungselement zu einer Führung des Elements entlang einer Zwangsbahn, insbesondere entlang der Punktionsrichtung. Vorzugsweise umfasst die Mechanikeinheit eine Antriebseinheit zu einer Erzeugung der Relativbewegung des Punktionselements. Vorzugsweise ist die Antriebseinheit elektromechanisch ausgebildet, insbesondere als Linearmotor oder Linearaktor. Alternativ kann ein Antrieb mechanisch, insbesondere mittels zumindest eines federelastischen Elements und/oder mittels einer Übersetzungseinheit, zu einer Übersetzung einer, insbesondere von einem Benutzer aufgewendeten, Kraft und/oder aufgewendeten Drehmoments auf das Punktionselement, ausgebildet sein. Vorzugsweise umfasst die Mechanikeinheit eine Ultraschalleinheit. Vorzugsweise ist die Ultraschalleinheit dazu vorgesehen, das von der Mechanikeinheit antreibbare Punktionselement der Punktionseinheit zu einer Schwingung anzuregen. Vorzugsweise ist zumindest während einer Punktion die durch die Mechanikeinheit hervorgerufene Relativbewegung des Punktionselements überlagert mit einer durch die Ultraschalleinheit hervorgerufene Schwingung des Punktionselements. In einer weiteren Ausgestaltung der Mechanikeinheit ist es denkbar, dass die Mechanikeinheit eine Ultraschalleinheit aufweist, welche zu einer unmittelbaren Punktion eines Körpers vorgesehen ist. Durch die erfindungsgemäße Ausgestaltung des handgehaltenen Messgeräts kann vorteilhaft ein Parameter der Punktion eingestellt werden. Insbesondere kann ein Kraftaufwand und eine Eindringtiefe des Elements der Punktionseinheit in den Körper auf ein notwendiges Minimum beschränkt werden. Insbesondere kann eine minimal invasive Punktion vorteilhaft zuverlässig und reproduzierbar gestaltet werden.

Weiterhin wird vorgeschlagen, dass die Punktionseinheit zur Punktion des Körpers eine Lasereinheit aufweist. Vorzugsweise umfasst die Lasereinheit zumindest eine Strahlquelleneinheit, insbesondere eine Laserdiode, zu einer Erzeugung eines Laserstrahls. Insbesondere erzeugt die Lasereinheit, insbesondere während der Punktion, einen Laserstrahl, der sich entlang der Punktionsrichtung ausbreitet. Vorzugsweise umfasst die Lasereinheit eine Optikeinheit zu einer Fokussierung des Laserstrahls, insbesondere auf einen an der Anlegefläche anliegenden Körper. Vorzugsweise ist die Lasereinheit dazu vorgesehen, mittels Laserablation einen Zugangskanal in den Körper zu erstellen. Insbesondere weist der zur Punktion vorgesehene Laserstrahl in einer zur Punktionsrichtung senkrechten Ebene einen maximalen Außendurchmesser von weniger als 1 mm, bevorzugt von weniger als 0,7 mm, besonders bevorzugt von weniger als 0,4 mm, auf. Durch die erfindungsgemäße Ausgestaltung des handgehaltenen Messgeräts kann vorteilhaft ein Parameter der Punktion eingestellt werden. Insbesondere kann eine Eindringtiefe des Elements der Punktionseinheit in den Körper auf ein notwendiges Minimum beschränkt werden. Insbesondere kann eine minimal invasive Punktion vorteilhaft zuverlässig und reproduzierbar gestaltet werden. Insbesondere kann ein Verschleiß gegenüber einer mechanischen Punktionseinheit verringert werden. Insbesondere kann ein Auswechseln des Punktionselements aus hygienischen Gründen vermieden werden.

Ferner wird vorgeschlagen, dass die Aufnahmeeinheit eine Unterdruckeinheit zur Erzeugung eines Unterdrucks, der zu einer Förderung der Körperflüssigkeit vorgesehen ist, aufweist. Vorzugsweise umfasst die Unterdruckeinheit eine Dichtungseinheit, die zumindest in einem zur Punktionsrichtung senkrechten Querschnitt die Punktionsrichtung, insbesondere das Punktionselement und/oder den Laserstrahl zur Punktion, sowie die Aufnahmeöffnung der Aufnahmeeinheit vollständig umschließt. Vorzugsweise schließt die Dichtungseinheit in der Anlegeebene mit einem, insbesondere elastischen, Anlegeelement ab. Vorzugsweise umgreift die Dichtungseinheit einen Bereich um die Aufnahmeöffnung der Aufnahmeeinheit herum, der zur Anlegeebene hin offen ist. Insbesondere ist die Dichtungseinheit dazu vorgesehen in einem an einem Körper mit dem Anlegeelement angeordneten Zustand des Messgeräts, einen Bereich um die Aufnahmeöffnung abzudichten. Vorzugsweise ist die Unterdruckeinheit dazu vorgesehen, einen Luftdruck des durch die Dichtungseinheit und den Körper eingeschlossenen Bereichs zu senken. Insbesondere ist die Unterdruckeinheit dazu vorgesehen, durch die Absenkung des Luftdrucks ein Austreten einer Körperflüssigkeit durch einen mittels der Punktionseinheit hergestellten Zugangskanal zu unterstützen. Bevorzugt wird aufgrund des Unterdrucks eine Menge der Körperflüssigkeit von zumindest 0,1 µl, besonders bevorzugt von zumindest 0,5 µl durch den mit der Punktionseinheit hergestellten Zugangskanal befördert. Bevorzugt wird aufgrund des Unterdrucks eine Menge der Körperflüssigkeit von 0,1 µl bis 15 µl, besonders bevorzugt von 0,5 µl bis 4 µl durch den mit der Punktionseinheit hergestellten Zugangskanal befördert. Bevorzugt umfasst die Dichtungseinheit ein Belüftungsventil, zu einer Wiederherstellung eines Normaldrucks im von der Dichtungseinheit eingeschlossenen Bereich. Durch die erfindungsgemäße Ausgestaltung des handgehaltenen Messgeräts kann trotz eines durch eine minimal-invasive Punktion erstellten Zugangskanals zur Körperflüssigkeit eine zu einer Analyse ausreichende Menge der Körperflüssigkeit gefördert werden.

Darüber hinaus wird vorgeschlagen, dass die Unterdruckeinheit zur Erzeugung eines Unterdrucks zumindest ein Heizelement aufweist, welches zumindest zu einer Temperaturerhöhung eines Bereichs an einer Aufnahmeöffnung eines Aufnahmeraums der Aufnahmeeinheit vorgesehen ist. Vorzugsweise ist das Heizelement als Heizstrahler ausgebildet. Bevorzugt ist das Heizelement an einer innenliegenden Wand der Dichtungseinheit angeordnet. Es ist auch denkbar, dass das Heizelement in eine Wand der Dichtungseinheit integriert ist. Vorzugsweise ist das Dichtungselement dazu vorgesehen einen Bereich mit einer erhöhten Temperatur abzudichten, insbesondere über das Schließen des Belüftungsventils. Alternativ kann der Bereich mit der erhöhten Temperatur auch durch ein Anbringen des handgehaltenen Messgeräts an den Körper abgedichtet werden. Vorzugsweise weist die Unterdruckeinheit eine Temperatursensoreinheit zu einer Regelung des Heizelements auf. Insbesondere weist die Unterdruckeinheit eine Schalteinheit zu einem Abschalten des Heizelements, insbesondere bei Erreichen einer vorgebbaren Temperatur, auf. Alternativ oder zusätzlich weist die Schalteinheit eine Zeitgebereinheit und/oder eine monostabile Kippstufe zu einer Steuerung einer Heizdauer auf. In einer alternativen Ausgestaltung des handgehaltenen Messgeräts, weist die Unterdruckeinheit eine Pumpeneinheit zur Erzeugen eines Unterdrucks eines Bereichs an einer Aufnahmeöffnung eines Aufnahmeraums der Aufnahmeeinheit auf. Durch die erfindungsgemäße Ausgestaltung des handgehaltenen Messgeräts kann vorteilhaft einfach ein Unterdruck zu einer Förderung einer Körperflüssigkeit durch einen durch eine minimal-invasive Punktion erzeugten Zugangskanal erzeugt werden.

Ferner wird vorgeschlagen, dass eine Füllstandausgabeeinheit zu einer Ausgabe eines Füllstandes der Aufnahmeeinheit vorgesehen ist. Vorzugsweise ist die Füllstandausgabeeinheit dazu vorgesehen, einen Füllstand des Aufnahmeraums der Aufnahmeeinheit mit einer Körperflüssigkeit auszugeben. Beispielsweise ist die Füllstandausgabeeinheit dazu vorgesehen kenntlich zu machen, dass eine zu einer Analyse ausreichende Menge einer Körperflüssigkeit sich in dem Aufnahmeraum befindet. Insbesondere macht die Füllstandausgabeeinheit kenntlich, dass sich zumindest mehr als 0,1 µl, bevorzugt mehr als 0,5 µl in dem Aufnahmeraum der Aufnahmeeinheit befinden. Beispielsweise ist die Füllstandausgabeeinheit dazu vorgesehen kenntlich zu machen, dass eine zu einer Analyse unzureichende Menge einer Körperflüssigkeit sich in dem Aufnahmeraum befindet. Vorzugsweise wird ein Füllstand elektronisch erfasst, insbesondere mittels einer kapazitiven Sensoreinheit der Füllstandausgabeeinheit. Es ist aber auch denkbar, dass zu einer Erfassung des Füllstands die Füllstandausgabeeinheit eine Ultraschalleinheit oder eine Durchstrahleinheit für elektromagnetische Strahlen, beispielsweise in Form zumindest einer Lichtschranke, aufweist. Eine Ausgabe des erfassten Füllstands kann visuell, akustisch und/oder haptisch erfolgen. Beispielsweise kann die Füllstandausgabeeinheit zu einer Ausgabe ein Display, Kontrollleuchten, Lautsprecher und/oder ein Vibrationselement aufweisen. Vorzugsweise wird bei einer unzureichenden Füllung für eine Analyse automatisch ein weiterer Unterdruckzyklus aktiviert zu einer zusätzlichen Beförderung von Körperflüssigkeit aus dem Körper. Alternativ oder zusätzlich kann die Füllstandausgabeeinheit ein in ein Gehäuse des handgehaltenen Messgeräts eingelassenes Sichtfenster aufweisen. Vorzugsweise ist das Sichtfenster dazu vorgesehen, eine Sicht von außen auf den Aufnahmeraum zu erlauben. Insbesondere weist der Aufnahmeraum an der einsehbaren Stelle eine durchsichtige Wandung und vorzugsweise ein Skalenelement, insbesondere eine Markierung eines minimal notwendigen Füllstands auf. Durch die erfindungsgemäße Ausgestaltung des handgehaltenen Messgeräts kann vorteilhaft eine Zuverlässigkeit der Aufnahme einer Körperflüssigkeit durch einen mittels einer minimal-invasiven Punktion erstellten Zugangskanal zu einer Körperflüssigkeit zumindest kontrolliert werden. Insbesondere kann eine hohe Zuverlässigkeit der Aufnahme einer Körperflüssigkeit durch einen mittels einer minimal-invasiven Punktion erstellten Zugangskanal zu einer Körperflüssigkeit durch eine elektronische Erfassung des Füllstands in Kombination mit einer Steuerung der Unterdruckeinheit erreicht werden.

Des Weiteren wird vorgeschlagen, dass die Aufnahmeeinheit eine, insbesondere einen Aufnahmeraum zumindest teilweise begrenzende, zumindest teilweise transparente Wandung zur Durchführung einer optischen Messung, insbesondere NIR-Spektroskopie, der Körperflüssigkeit aufweist. Bevorzugt weist das handgehaltene Messgerät eine Optikmesseinheit auf. Vorzugsweise ist die Optikmesseinheit dazu vorgesehen eine optische Messung durchzuführen. Vorzugsweise ist die Optikmesseinheit dazu vorgesehen Nahinfrarot (NIR)-Spektroskopie durchzuführen. Unter einer "zumindest teilweise transparente Wandung zur Durchführung einer optischen Messung" soll insbesondere eine Wandung verstanden werden, die aus einem Material besteht, welches zumindest bei einer Mittenfrequenz der in der optischen Messung verwendeten elektromagnetischen Strahlung einen Transmissionsgrad von zumindest 50 %, bevorzugt zumindest 75 % aufweist. Insbesondere begrenzt die zumindest teilweise transparente Wandung zumindest teilweise den Aufnahmeraum. Vorzugsweise gewährt die teilweise transparente Wandung eine Wechselwirkung einer in der optischen Messung verwendeten, insbesondere außerhalb der Aufnahmeeinheit erzeugten, elektromagnetischen Strahlung mit einer sich in dem Aufnahmeraum befindlichen Körperflüssigkeit. Durch die erfindungsgemäße Ausgestaltung des handgehaltenen Messgeräts können vorteilhaft zusätzliche Messdaten aus einer einzigen Probe einer Körperflüssigkeit gewonnen werden. Insbesondere kann eine Entnahme einer weiteren Probe der Körperflüssigkeit vermieden werden.

Weiter wird vorgeschlagen, dass die Aufnahmeeinheit zumindest ein beweglich gelagertes Aufnahmeelement zur Aufnahme der Körperflüssigkeit umfasst. Insbesondere umfasst das Aufnahmeelement den Aufnahmeraum zur Aufnahme der Körperflüssigkeit. Vorzugsweist umfasst die Aufnahmeeinheit ein Führungselement zu einer Führung des beweglich gelagerten Aufnahmeelements. Vorzugsweise umfasst die Aufnahmeeinheit eine Antriebseinheit zu einer Durchführung einer Bewegung des beweglich gelagerten Aufnahmeelements. Vorzugsweise ist die Antriebseinheit dazu vorgesehen, das beweglich gelagerte Aufnahmeelement in verschiedene Funktionsbereiche des handgehaltenen Messgeräts zu transportierten. Beispielsweise ist das beweglich gelagerte Aufnahmeelement dazu vorgesehen, aus einem Aufnahmebereich zur Aufnahme der Körperflüssigkeit in einen Analysebereich zur Erfassung der Kenngröße der aufgenommenen Körperflüssigkeit bewegt zu werden. Es ist auch denkbar, dass ein Abstand des Aufnahmeelements zur Anlegeebene, insbesondere zu einem anliegenden Körper, zu einer Aufnahme der Körperflüssigkeit gesteuert und/oder geregelt wird. Alternativ oder zusätzlich ist das Aufnahmeelement über ein federelastisches Element zu einem Abfangen einer in Punktionsrichtung wirkenden Kraft auf das Aufnahmeelement, insbesondere bei einem Anlegen des handgehaltenen Messgeräts an einen Körper, gelagert. Durch die erfindungsgemäße Ausgestaltung des handgehaltenen Messgeräts kann vorteilhaft eine hohe Zuverlässigkeit der Aufnahme einer Körperflüssigkeit durch einen mittels einer minimal-invasiven Punktion erstellten Zugangskanal zu einer Körperflüssigkeit durch eine Abstandsanpassung erreicht werden. Insbesondere können vorteilhaft verschiedene Bereiche, beispielsweise zu verschiedenen Messungen, räumlich getrennt werden, um eine gegenseitige Beeinträchtigung zu minimieren.

Ferner wird vorgeschlagen, dass die Aufnahmeeinheit zumindest zwei, insbesondere im Wesentlichen senkrecht zur Punktionsrichtung, beweglich gelagerte Aufnahmeelemente, welche zur Aufnahme der Körperflüssigkeit, insbesondere innerhalb, der Aufnahmeeinheit austauschbar sind aufweist. Insbesondere umfasst ein Aufnahmeelement einen Aufnahmeraum zur Aufnahme der Körperflüssigkeit. Insbesondere sind verschiedene Aufnahmeelemente dazu vorgesehen, bei verschiedenen Messungen mit dem handgehaltenen Messgerät die Körperflüssigkeit aufzunehmen. Vorzugsweise umfasst die Aufnahmeeinheit mehr als 5, bevorzugt mehr als 15, besonders bevorzugt mehr als 30 Aufnahmeelemente. Vorzugsweise umfasst die Aufnahmeeinheit eine Magazineinheit zu einer Aufbewahrung nicht verwendeter Aufnahmeelemente. Vorzugsweise ist die Magazineinheit als Trommelmagazin ausgebildet. Vorzugsweise ist eine Drehachse des Trommelmagazins im Wesentlichen parallel zur Punktionsrichtung angeordnet. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Es ist aber auch denkbar, dass die Magazineinheit als Kasten- oder Stangenmagazin ausgebildet ist. Vorzugsweise ist die Magazineinheit, insbesondere werkzeuglos, zerstörungsfrei lösbar an einem Gehäuse und/oder einer Halteeinheit innerhalb des Gehäuses des handgehaltenen Messgeräts angeordnet, insbesondere zu einem Austausch des Magazins durch einen Benutzer. Durch die erfindungsgemäße Ausgestaltung des handgehaltenen Messgeräts kann vorteilhaft ein hoher Benutzerkomfort erreicht werden. Insbesondere können mehrere Messungen vorgenommen werden, bevor ein Austausch eines sich in dem handgehaltenen Messgerät befindlichen, insbesondere gefüllten, Aufnahmeelements mit einem sich außerhalb des handgehaltenen Messgeräts befindlichen, insbesondere leeren, Aufnahmeelement, insbesondere durch einen Benutzer, vorgenommen werden muss.

Des Weiteren wird vorgeschlagen, dass die Punktionseinheit zumindest zwei, insbesondere im Wesentlichen senkrecht zur Punktionsrichtung, beweglich gelagerte Punktionselemente aufweist, welche insbesondere gemeinsam mit beweglich gelagerten Aufnahmeelementen der Aufnahmeeinheit austauschbar sind. Insbesondere sind verschiedene Punktionselemente dazu vorgesehen, bei verschiedenen Punktionen mit dem handgehaltenen Messgerät eingesetzt zu werden. Vorzugsweise umfasst die Aufnahmeeinheit mehr als 5, bevorzugt mehr als 15, besonders bevorzugt mehr als 30 Punktionselemente. Vorzugsweise umfasst die Aufnahmeeinheit eine Punktionsmagazineinheit zu einer Aufbewahrung nicht verwendeter Punktionselemente. Vorzugsweise ist die Punktionsmagazineinheit als Trommelmagazin ausgebildet. Vorzugsweise ist eine Drehachse des Trommelmagazins im Wesentlichen parallel zur Punktionsrichtung angeordnet. Es ist aber auch denkbar, dass die Punktionsmagazineinheit als Kasten- oder Stangenmagazin ausgebildet ist. Vorzugsweise ist die Punktionsmagazineinheit, insbesondere werkzeuglos, zerstörungsfrei lösbar an einem Gehäuse und/oder einer Halteeinheit innerhalb des Gehäuses des handgehaltenen Messgeräts angeordnet, insbesondere zu einem Austausch der Punktionsmagazineinheit durch einen Benutzer. Besonders bevorzugt ist die Punktionsmagazineinheit einstückig oder zumindest form- und/oder kraftschlüssig mit der Magazineinheit der Aufnahmeeinheit ausgebildet. Insbesondere ist es auch denkbar, dass die Punktionselemente einstückig oder zumindest form- und/oder kraftschlüssig mit je einem Aufnahmeelement ausgebildet sind, insbesondere zu einer Lagerung in einer gemeinsamen Magazineinheit. Es ist auch denkbar, dass die Punktionsmagazineinheit und die Magazineinheit unabhängig voneinander beweglich und/oder zerstörungsfrei lösbar gelagert sind. Durch die erfindungsgemäße Ausgestaltung des handgehaltenen Messgeräts kann vorteilhaft ein hoher Benutzerkomfort erreicht werden. Insbesondere können mehrere Messungen vorgenommen werden, bevor ein Austausch eines sich in dem handgehaltenen Messgerät befindlichen, insbesondere teilweise mit einer Körperflüssigkeit benetztes, Punktionselement mit einem sich außerhalb des handgehaltenen Messgeräts befindlichen, insbesondere sauberen, Punktionselement, insbesondere durch einen Benutzer, vorgenommen werden muss.

Weiterhin wird ein Verfahren zu einem Betrieb eines erfindungsgemäßen handgehaltenen Messgeräts zur Analyse von Körperflüssigkeiten, insbesondere von Blut, vorgeschlagen. Erfindungsgemäß umfasst das Verfahren zumindest einen Verfahrensschritt zu einer minimal-invasiven Punktion eines Körpers mittels der Punktionseinheit des handgehaltenen Messgeräts; einen Verfahrensschritt, in dem eine aus dem Körper aufgrund der Punktion austretende Körperflüssigkeit durch die Aufnahmeeinheit aufgenommen wird, insbesondere unter Nutzung eines Unterdrucks; und einen Verfahrensschritt zur Erfassung von Messsignalen der in der Aufnahmeeinheit befindlichen Körperflüssigkeit mittels der Kernspinresonanz-Sensoreinheit.

Vorzugsweise werden die Verfahrensschritte automatisch nacheinander und/oder parallel zueinander ausgeführt, insbesondere mittels einer zentralen Steuer- und/oder Regeleinheit, insbesondere zumindest im Wesentlichen frei von einem Benutzereingriff. Durch die erfindungsgemäße Ausgestaltung des Verfahrens kann ein komfortabler Betrieb des handgehaltenen Messgeräts realisiert werden, der eine vorteilhaft komfortable Entnahme einer Körperflüssigkeit ermöglicht.

Das erfindungsgemäße handgehaltene Messgerät und/oder das erfindungsgemäße Verfahren sollen/soll hierbei nicht auf die oben beschriebene Anwendung und Ausführungsform beschränkt sein. Insbesondere können/kann das erfindungsgemäße handgehaltene Messgerät und/oder das erfindungsgemäße Verfahren zu einer Erfüllung einer hierin beschriebenen Funktionsweise eine von einer hierin genannten Anzahl von einzelnen Elementen, Bauteilen, Verfahrensschritten und Einheiten abweichende Anzahl aufweisen, sofern dies in den durch die Ansprüche definierten Schutzumfang fällt. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind drei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, sofern dies in den durch die Ansprüche definierten Schutzumfang fällt.

Es zeigen:
- Fig. 1: eine schematische Darstellung eines erfindungsgemäßen handgehaltenen Messgeräts mit einem Punktionselement zur Punktion,
- Fig. 2: ein Flussdiagramm eines erfindungsgemäßen Verfahrens zu einem Betrieb des erfindungsgemäßen handgehaltenen Messgeräts,
- Fig. 3: eine schematische Darstellung eines alternativen erfindungsgemäßen handgehaltenen Messgeräts mit einer Lasereinheit zur Punktion,
- Fig. 4: eine schematische Darstellung eines weiteren alternativen erfindungsgemäßen handgehaltenen Messgeräts mit einem Trommelmagazin und
- Fig. 5: eine schematische Darstellung eines weiteren alternativen erfindungsgemäßen handgehaltenen Messgeräts mit einem Punktionsmagazin.

### Beschreibung der Ausführungsbeispiele

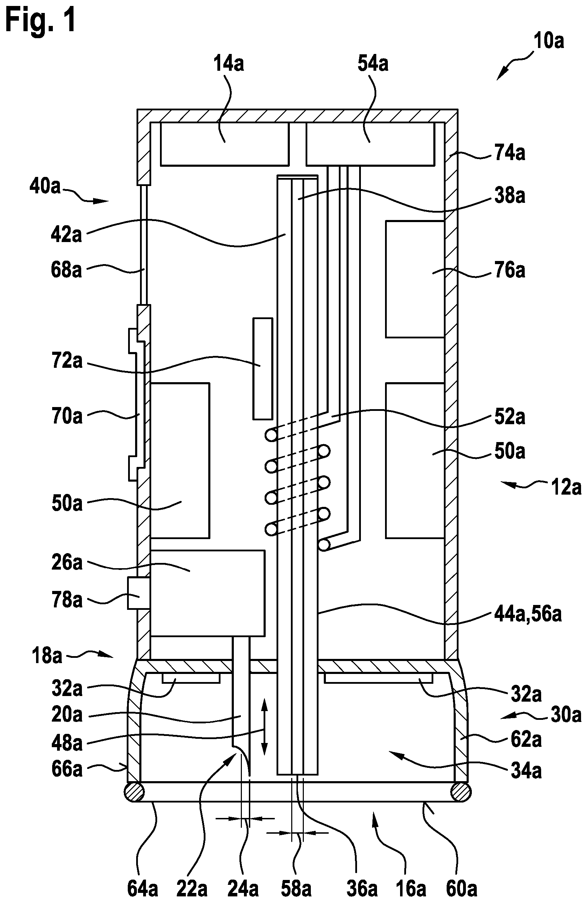

Figur 1 zeigt eine schematische Darstellung eines handgehaltenen Messgeräts 10a zur Analyse von Körperflüssigkeiten, insbesondere von Blut, mit zumindest einer Kernspinresonanz-Sensoreinheit 12a. Vorzugsweise umfasst die Kernspinresonanz-Sensoreinheit 12a, zumindest eine Magnetfeldeinheit 50a zur Erzeugung eines statischen Magnetfeldes. Vorzugsweise ist die Magnetfeldeinheit 50a als Permanentmagnet ausgebildet. Vorzugsweise weist die Kernspinresonanz-Sensoreinheit 12a eine Induktionsspule 52a zu einem Aussenden und/oder Empfangen eines magnetischen Wechselfeldes auf. Bevorzugt weist die Kernspinresonanz-Sensoreinheit 12a eine Steuereinheit 54a zu einer Durchführung eines spektroskopischen Verfahrens auf. Das handgehaltene Messgerät 10a weist zumindest eine Auswerteeinheit 14a zur Auswertung eines von der Kernspinresonanz-Sensoreinheit 12a gelieferten Messsignals auf. Das handgehaltene Messgerät 10a umfasst zumindest eine Aufnahmeeinheit 16a, insbesondere eine Mikrokapillareinheit, zur Aufnahme der Körperflüssigkeit. Vorzugsweise weist die Aufnahmeeinheit 16a zumindest ein Aufnahmeelement 44a mit einem Aufnahmeraum 38a zur Aufnahme der Körperflüssigkeit auf. Insbesondere ist das Aufnahmeelement 44a als Kapillare 56a, insbesondere als Mikrokapillare, ausgebildet. Bevorzugt weist die Kapillare 56a in einer zu einer maximalen Erstreckung einer inneren Mantelfläche der Kapillare 56a senkrechten Ebene einen größten Innendurchmesser 58a zwischen 0,05 und 0,5 mm, besonders bevorzugt zwischen 0,1 und 0,3 mm, auf. Vorzugsweise weist der Aufnahmeraum 38a eine Aufnahmeöffnung 36a auf. Bevorzugt gelangt die Körperflüssigkeit durch die Aufnahmeöffnung 36a, insbesondere aufgrund einer Kapillarkraft, in den Aufnahmeraum 38a. Vorzugsweise weist das handgehaltene Messgerät 10a eine Anlegefläche 60a auf, die dazu vorgesehen ist an einen Körper angelegt zu werden. Das handgehaltene Messgerät 10a umfasst zumindest eine Punktionseinheit 18a zur minimal-invasiven Punktion des Körpers, insbesondere eines Kapillarbetts des Körpers. Die Punktionseinheit 18a weist zumindest ein Punktionselement 20a, insbesondere eine Lanzette, auf, welches zumindest in einem zur Punktion vorgesehenen Bereich 22a einen maximalen Außendurchmesser 24a von weniger als 1 mm aufweist. Vorzugsweise handelt es sich bei dem Punktionselement 20a um ein Stechwerkzeug, insbesondere um eine Lanzette. Alternativ kann das Punktionselement 20a auch zu einer gleichzeitigen Aufnahme einer Körperflüssigkeit einstückig mit der Aufnahmeeinheit 16a ausgebildet sein, beispielsweise als Kanüle. Das Punktionselement 20a kann einteilig mit dem Aufnahmeelement 44a der Aufnahmeeinheit 16a ausgebildet sein. Die Punktionseinheit 18a weist zur Punktion des Körpers eine, insbesondere ultraschallgetriebene, Mechanikeinheit 26a auf. Vorzugsweise umfasst die Mechanikeinheit 26a einen elektrostatischen, elektromagnetischen und/oder magnetostriktiven Linearaktor zu einer Erzeugung einer Relativbewegung eines Punktionselements 20a. Vorzugsweise umfasst die Mechanikeinheit 26a einen Impulsgenerator zu einer Steuerung des Linearaktors. Vorzugsweise ist das Punktionselement 20a zerstörungsfrei lösbar mit dem Linearaktor verbunden. Insbesondere kann das Punktionselement 20a, insbesondere werkzeuglos, durch einen Benutzer ausgetauscht werden. Alternativ weist die Mechanikeinheit 26a ein federelastisches Antriebselement auf.

Die Aufnahmeeinheit 16a weist eine Unterdruckeinheit 30a zur Erzeugung eines Unterdrucks, der zur Förderung der Körperflüssigkeit vorgesehen ist, auf.

Vorzugsweise umfasst die Unterdruckeinheit 30a eine Dichtungseinheit 62a, die zumindest in einem zur Punktionsrichtung 48a senkrechten Querschnitt das Punktionselement 20a sowie die Aufnahmeöffnung 36a der Aufnahmeeinheit 16a vollständig umschließt. Vorzugsweise schließt die Dichtungseinheit 62a in der Anlegeebene mit einem, insbesondere elastischen, Anlegeelement 64a ab. Vorzugsweise umgreift die Dichtungseinheit 62a einen Bereich 34a um die Aufnahmeöffnung 36a der Aufnahmeeinheit 16a herum, der zur Anlegefläche 60a hin offen ist. Vorzugsweise weist die Dichtungseinheit 62a ein Sichtschutzelement 66a auf und/oder ist aus einem undurchsichtigen Material gefertigt, um eine Beobachtung einer austretenden Körperflüssigkeit, insbesondere durch unbeteiligte Personen, zu vermeiden. Die Unterdruckeinheit 30a weist zur Erzeugung eines Unterdrucks zumindest ein Heizelement 32a auf, welches zumindest zu einer Temperaturerhöhung des Bereichs 34a an der Aufnahmeöffnung 36a des Aufnahmeraums 38a der Aufnahmeeinheit 16a vorgesehen ist.

Das handgehaltenes Messgerät 10a umfasst eine Füllstandausgabeeinheit 40a zu einer Ausgabe eines Füllstandes der Aufnahmeeinheit 16a. Bevorzugt umfasst das Messgerät 10a ein Sichtfenster 68a zu einer Sicht auf den Aufnahmeraum 38a durch eine transparente Wandung 42a der Kapillare 56a. Vorzugsweise umfasst das handgehaltene Messgerät 10a ein Display 70a zur Anzeige einer Auswertung der Auswerteeinheit 14a. Vorzugsweise ist das Display 70a dazu vorgesehen, einen Füllstand der Aufnahmeeinheit 16a anzuzeigen. Vorzugsweise wird ein Füllstand des Aufnahmeraums 38a mittels einer kapazitiven Sensoreinheit 72a erfasst.

Vorzugsweise umfasst das handgehaltene Messgerät 10a ein Gehäuse 74a. Vorzugsweise ist das Gehäuse 74a zu einem Schutz zumindest des Aufnahmeelements 44a vorgesehen. Bevorzugt umfasst das Messgerät 10a über zumindest eine, insbesondere zentrale, Steuer- und/oder Regeleinheit 76a mit einer Prozessoreinheit und mit einer Speichereinheit sowie mit einem in der Speichereinheit gespeicherten Betriebsprogramm zur Durchführung eines Verfahren 98a zu einem Betrieb des handgehaltenen Messgeräts 10a. Vorzugsweise führt das Betriebsprogramm nach einem Einschalten des handgehaltenen Messgeräts 10a eine Selbstdiagnose durch.

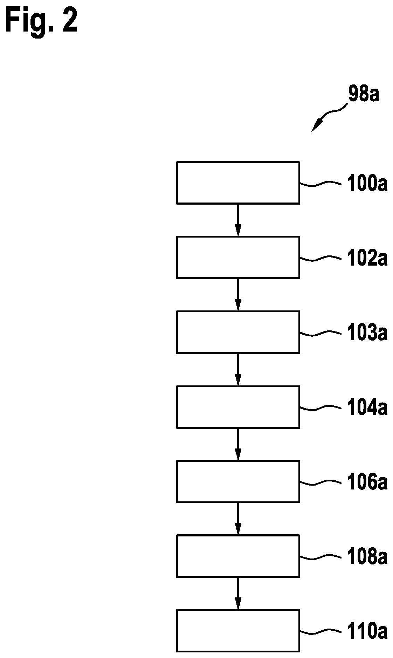

Figur 2 zeigt ein Flussdiagramm des Verfahrens 98a zu einem Betrieb des handgehaltenen Messgeräts zur Analyse von Körperflüssigkeiten, insbesondere Blut. Vorzugsweise umfasst das handgehaltene Messgerät 10a zumindest ein Bedienelement 78a zu einem Auslösen des Verfahrens 98a. Optional weist das Messgerät 10a weitere Bedienelemente, insbesondere zu einer Einstellung von Betriebsparametern auf. Bevorzugt wird das handgehaltene Messgerät 10a von einem Benutzer auf einen Körper aufgesetzt und anschließend das Verfahren durch eine Betätigung 100a des Bedienelements 78a gestartet. Vorzugsweise wird ein Unterdruck in dem Bereich 34a erzeugt. Bevorzugt wird nach der Erzeugung des Unterdrucks 102a, ein Punktionsverfahrensschritt 103a durchgeführt. Insbesondere sticht während des Punktionsverfahrensschritts 103a das Punktionselement 20a in den Körper und wird anschließend zurückgezogen. Vorzugsweise wird während eines Aufnahmeverfahrensschritts 104a eine austretende Menge der Körperflüssigkeit mit der Aufnahmeeinheit 16a, insbesondere selbsttätig, in den Aufnahmeraum 38a aufgenommen. Vorzugsweise wird eine Kontrolle 106a des Füllstands des Aufnahmeraums 38a mit einer Körperflüssigkeit durchgeführt. Vorzugsweise wird bei einem ausreichenden Füllstands des Aufnahmeraums 38a, einem Benutzer mittels der Füllstandausgabeeinheit 40a mitgeteilt, dass das handgehaltene Messgerät 10a vom Körper entfernt werden kann. Insbesondere wird ein Messverfahren 108a gestartet. Insbesondere wird ein Kernspinresonanz-Spektrum erstellt. Bevorzugt werden die während des Messverfahrens 108a erzeugten Messsignale in der Auswerteeinheit 14a ausgewertet. Vorzugsweise wird in einem Ausgabeverfahrensschritt 110a eine ermittelte Kenngröße der Körperflüssigkeit auf dem Display 70a ausgegeben. Optional wird die Kenngröße der Körperflüssigkeit während des Ausgabeverfahrensschritts 110a an ein externes Gerät (hier nicht näher dargestellt) übermittelt.

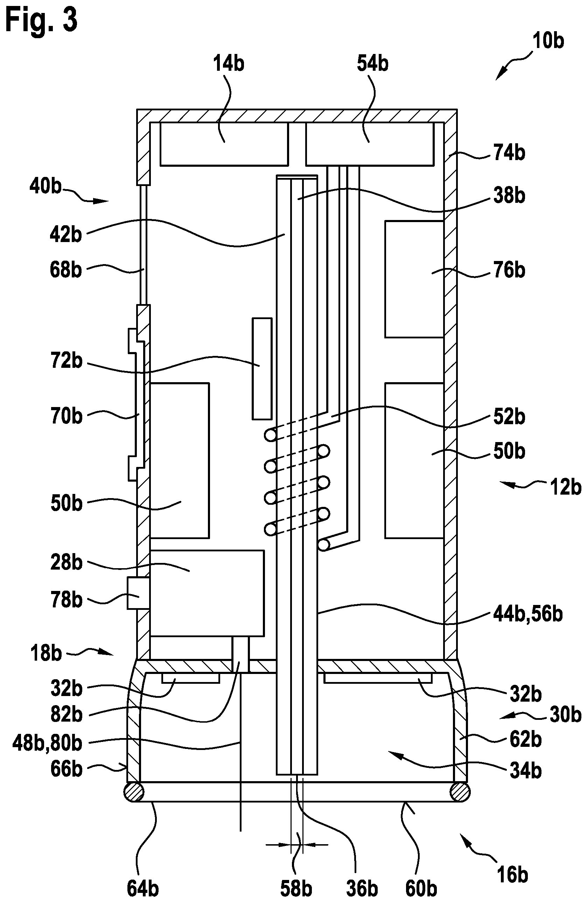

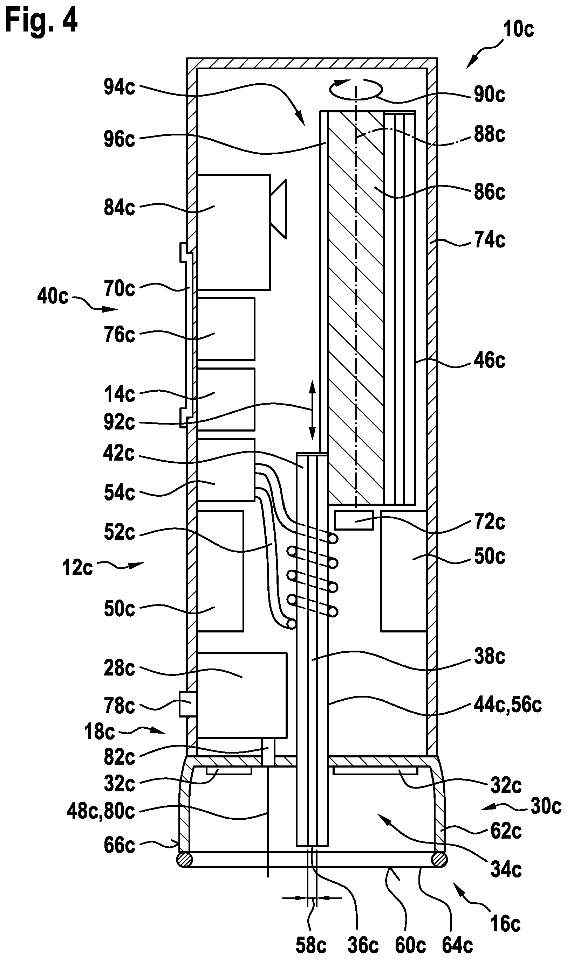

In den Figuren 3 und 4 ist je ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 und 2, verwiesen werden kann. Zur Unterscheidung der Ausführungsbeispiele ist der Buchstabe a den Bezugszeichen des Ausführungsbeispiels in den Figuren 1 und 2 nachgestellt. In den Ausführungsbeispielen der Figuren 3 und 4 ist der Buchstabe a durch die Buchstaben b oder c ersetzt.

Figur 3 zeigt eine schematische Darstellung eines handgehaltenen Messgeräts 10b zur Analyse von Körperflüssigkeiten, insbesondere Blut, mit zumindest einer Kernspinresonanz-Sensoreinheit 12b. Das handgehaltene Messgerät 10b weist zumindest eine Auswerteeinheit 14b zur Auswertung eines von der Kernspinresonanz-Sensoreinheit 12b gelieferten Messsignals auf. Das handgehaltene Messgerät 10b umfasst zumindest eine Aufnahmeeinheit 16b, insbesondere eine Mikrokapillareinheit, zur Aufnahme der Körperflüssigkeit. Das handgehaltene Messgerät 10b umfasst zumindest eine Punktionseinheit 18b zur minimal-invasiven Punktion des Körpers, insbesondere eines Kapillarbetts des Körpers. Die Punktionseinheit 18b weist zur Punktion des Körpers eine Lasereinheit 28b auf. Insbesondere erzeugt die Lasereinheit 28b, insbesondere während des Punktionsverfahrensschritts 103b, einen Laserstrahl 80b, der sich entlang der Punktionsrichtung 48b ausbreitet. Vorzugsweise umfasst die Lasereinheit 28b eine Optikeinheit 82b zu einer Fokussierung des Laserstrahls 80b. Vorzugsweise weist die Lasereinheit 28b zumindest eine Sicherheitseinheit auf. Vorzugsweise ist die Sicherheitseinheit dazu vorgesehen zu verhindern, dass ein Laserstrahl 80b sich ausgehend von der Lasereinheit 28b über die Anlegefläche 60b hinaus ausbreiten kann. Insbesondere umfasst die Sicherheitseinheit ein Schaltelement um den Laserstrahl 80b abzublocken und/oder eine Erzeugung des Laserstrahls 80b zu unterbrechen, insbesondere in Abhängigkeit von einem Sicherheitsparameter. Beispielsweise könnte die Lasereinheit 28b einen Helligkeitssensor umfassen, der eine Lichtmenge in dem Bereich 34b misst. Beispielsweise könnte die Lasereinheit 28b einen Laserdetektor und eine Laserauswerteeinheit umfassen, zu einer Auswertung eines zurückgestreuten Teils des Laserstrahls 80b bezüglich einer zurückgelegten Distanz. Beispielsweise könnte an der Dichtungseinheit 62b, insbesondere an dem Anlegeelement 64b, ein Annäherungssensor zu einer Erfassung einer Anwesenheit eines Körpers angeordnet sein. Hinsichtlich weiterer Merkmale und/oder Funktionen des handgehaltenen Messgeräts 10b darf auf die Beschreibung der Figuren 1 und 2 verwiesen werden.

Figur 4 zeigt eine schematische Darstellung eines handgehaltenen Messgeräts 10c zur Analyse von Körperflüssigkeiten, insbesondere Blut, mit zumindest einer Kernspinresonanz-Sensoreinheit 12c. Das handgehaltene Messgerät 10c weist zumindest eine Auswerteeinheit 14c zur Auswertung eines von der Kernspinresonanz-Sensoreinheit 12c gelieferten Messsignals auf. Das handgehaltene Messgerät 10c umfasst zumindest eine Aufnahmeeinheit 16c, insbesondere eine Mikrokapillareinheit, zur Aufnahme der Körperflüssigkeit. Das handgehaltene Messgerät 10c umfasst zumindest eine Punktionseinheit 18c zur minimal-invasiven Punktion des Körpers, insbesondere eines Kapillarbetts des Körpers.

Die Aufnahmeeinheit 16c weist eine, insbesondere einen Aufnahmeraum 38c zumindest teilweise begrenzende, zumindest teilweise transparente Wandung 42c zur Durchführung einer optischen Messung, insbesondere NIR-Spektroskopie, der Körperflüssigkeit auf. Bevorzugt weist das handgehaltene Messgerät 10c eine Optikmesseinheit 84c auf. Vorzugsweise ist die Optikmesseinheit 84c dazu vorgesehen ein NIR-Spektrum der sich in dem Aufnahmeraum 38c befindlichen Körperflüssigkeit zu erfassen. Vorzugsweise werden Messdaten der Optikmesseinheit 84c in der Auswerteeinheit 14c ausgewertet.

Die Aufnahmeeinheit 16c umfasst zumindest ein beweglich gelagertes Aufnahmeelement 44c, 46c zur Aufnahme der Körperflüssigkeit. Die Aufnahmeeinheit 16c umfasst zumindest zwei, insbesondere im Wesentlichen senkrecht zur Punktionsrichtung 48c, beweglich gelagerte Aufnahmeelemente 44c, 46c, welche zur Aufnahme der Körperflüssigkeit, insbesondere innerhalb der Aufnahmeeinheit, 16c austauschbar sind. Vorzugsweise umfasst die Aufnahmeeinheit 16c eine als Trommelmagazin ausgebildete Magazineinheit 86c zu einer Aufbewahrung nicht verwendeter Aufnahmeelemente 46c. Vorzugsweise ist eine Drehachse 88c des Trommelmagazins im Wesentlichen parallel zur Punktionsrichtung 48c angeordnet. Vorzugsweise ist das Trommelmagazin zu einer, insbesondere schrittweisen, Drehung 90c um die Drehachse 88c mittels einer, insbesondere elektrischen, Drehantriebseinheit der Aufnahmeeinheit 16c antreibbar. Es ist aber auch vorstellbar, dass die Magazineinheit 86c manuell, insbesondere von einem Benutzer, drehbar ist. Vorzugsweise rastet ein Aufnahmeelement 44c in eine Aufnahme-und/oder Messposition 94c der Drehung 90 ein. Vorzugsweise umfasst die Aufnahmeeinheit 16c, insbesondere die Magazineinheit 86c ein Führungselement 96c zu einer Führung des beweglich gelagerten Aufnahmeelements 44c entlang einer Bewegungsrichtung 92c. Vorzugsweise umfasst die Aufnahmeeinheit 16c eine Antriebseinheit, zu einer Bewegung eines Aufnahmeelements 44c, welches sich in der Aufnahme-und/oder Messposition 94c befindet. Vorzugsweise wird das Aufnahmeelement 44c zu einer Aufnahme einer Körperflüssigkeit zur Anlegefläche 60c hinbewegt. Vorzugsweise wird das Aufnahmeelement 44c zu einer Analyse der Körperflüssigkeit mittels der Optikmesseinheit 84c und/oder einem Austausch der Aufnahmeelemente 44c, 46c von der Anlegefläche 60c wegbewegt. Vorzugsweise umfasst die Aufnahmeeinheit 16c eine Antriebseinheit zu einer Durchführung einer Bewegung des beweglich gelagerten Aufnahmeelements 44c. Hinsichtlich weiterer Merkmale und/oder Funktionen des handgehaltenen Messgeräts 10c darf auf die Beschreibung der Figuren 1 bis 3 verwiesen werden.

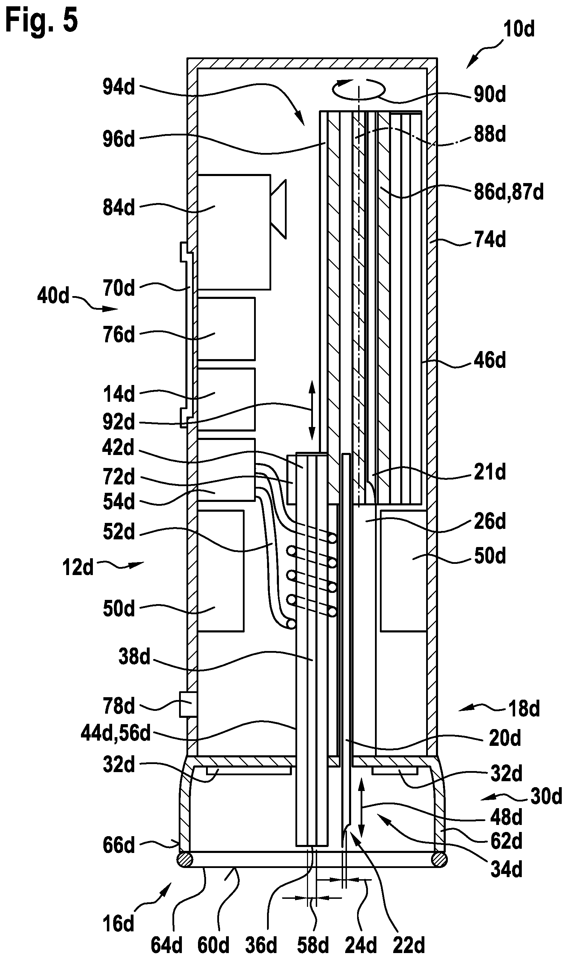

Figur 5 zeigt eine schematische Darstellung eines handgehaltenen Messgeräts 10d zur Analyse von Körperflüssigkeiten, insbesondere Blut, mit zumindest einer Kernspinresonanz-Sensoreinheit 12d. Das handgehaltene Messgerät 10d weist zumindest eine Auswerteeinheit 14d zur Auswertung eines von der Kernspinresonanz-Sensoreinheit 12d gelieferten Messsignals auf. Das handgehaltene Messgerät 10d umfasst zumindest eine Aufnahmeeinheit 16d, insbesondere eine Mikrokapillareinheit, zur Aufnahme der Körperflüssigkeit. Das handgehaltene Messgerät 10d umfasst zumindest eine Punktionseinheit 18d zur minimal-invasiven Punktion des Körpers, insbesondere eines Kapillarbetts des Körpers.

Die Punktionseinheit 18d weist zumindest ein Punktionselement 20d, 21d auf, das einteilig mit einem Aufnahmeelement 44d, 46d der Aufnahmeeinheit 16d ausgebildet ist. Bevorzugt ist jeweils eines der Punktionselemente 20d, 21d einteilig mit einem Aufnahmeelement 44d, 46d ausgebildet. Es ist jedoch auch denkbar, dass das Punktionselement 20d, 21d lösbar an einem Aufnahmeelement 44d, 46d angeordnet ist, wie beispielsweise mittels einer form- und/oder kraftschlüssigen Verbindung zwischen dem Punktionselement 20d, 21d und dem Aufnahmeelement 44d, 46d.

Die Punktionseinheit 18d weist zumindest zwei, insbesondere im Wesentlichen senkrecht zur Punktionsrichtung 48d, beweglich gelagerte Punktionselemente 20d, 21d auf, welche insbesondere gemeinsam mit beweglich gelagerten Aufnahmeelementen 44d, 46d der Aufnahmeeinheit 16d austauschbar sind. Insbesondere sind die Punktionselemente 44d, 46d in und/oder an einer Punktionsmagazineinheit 87d, insbesondere einem Trommelmagazin, angeordnet. Vorzugsweise ist die Punktionsmagazineinheit 87d für die Punktionselemente 20d, 21d analog ausgebildet zur oben beschriebenen Magazineinheit 86c für die Aufnahmeelemente 44c, 46c. Vorzugsweise ist die Punktionsmagazineinheit 87d einteilig mit der Magazineinheit 86d ausgebildet. Vorzugsweise umfasst das handgehaltene Messgerät 10d eine gemeinsame Magazineinheit 86d zur Lagerung der Aufnahmeelemente 44d, 46d und der Punktionselemente 20d, 21d. Vorzugsweise kann in der Punktionsmagazineinheit 87d pro Aufnahmeelement 44d, 46d zumindest ein Punktionselement 20d, 21d gelagert werden. Hinsichtlich weiterer Merkmale und/oder Funktionen des handgehaltenen Messgeräts 10d darf auf die Beschreibung der Figuren 1 bis 4 verwiesen werden.

## Patentansprüche

1. Handgehaltenes Messgerät ausgebildet zur Analyse von Körperflüssigkeiten, insbesondere Blut, mit zumindest einer Kernspinresonanz-Sensoreinheit (12a; 12b; 12c; 12d), mit zumindest einer Auswerteeinheit (14a; 14b; 14c; 14d) ausgebildet zur Auswertung eines von der Kernspinresonanz-Sensoreinheit (12a; 12b; 12c; 12d) gelieferten Messsignals und mit zumindest einer Aufnahmeeinheit (16a, 16b; 16c; 16d), insbesondere Mikrokapillareinheit, ausgebildet zur Aufnahme der Körperflüssigkeit, **gekennzeichnet durch** zumindest eine Punktionseinheit (18a; 18b; 18c; 18d) ausgebildet zur minimal-invasiven Punktion eines Körpers, insbesondere eines Kapillarbetts eines Körpers.

2. Handgehaltenes Messgerät nach Anspruch 1, **dadurch gekennzeichnet, dass** die Punktionseinheit (18a; 18d) zumindest ein Punktionselement (20a; 20d, 21d), insbesondere eine Lanzette, aufweist, welches zumindest in einem zur Punktion vorgesehenen Bereich (22a; 22d) einen maximalen Außendurchmesser (24a; 24d) von weniger als 1 mm aufweist.

3. Handgehaltenes Messgerät nach Anspruch 1 oder 2, **dadurch gekennzeichnet, dass** die Punktionseinheit (18a; 18d) zur Punktion des Körpers eine, insbesondere ultraschallgetriebene, Mechanikeinheit (26a; 26d) aufweist.

4. Handgehaltenes Messgerät nach einem der vorhergehenden Ansprüche, **dadurch gekennzeichnet, dass** die Punktionseinheit (18b; 18c) zur Punktion des Körpers eine Lasereinheit (28b; 28c) aufweist.

5. Handgehaltenes Messgerät nach einem der vorhergehenden Ansprüche, **dadurch gekennzeichnet, dass** die Aufnahmeeinheit (16a; 16b; 16c; 16d) eine Unterdruckeinheit (30a; 30b; 30c; 30d) zur Erzeugung eines Unterdrucks, der zu einer Förderung der Körperflüssigkeit vorgesehen ist, aufweist.

6. Handgehaltenes Messgerät nach Anspruch 5, **dadurch gekennzeichnet, dass** die Unterdruckeinheit (30a; 30b; 30c; 30d) zur Erzeugung eines Unterdrucks zumindest ein Heizelement (32a; 32b; 32c; 32d) aufweist, welches zumindest zu einer Temperaturerhöhung eines Bereichs (34a; 34b; 34c; 34d) an einer Aufnahmeöffnung (36a; 36b; 36c; 38d) eines Aufnahmeraums (38a; 38b; 38c; 38d) der Aufnahmeeinheit (16a; 16b; 16c; 16d) vorgesehen ist.

7. Handgehaltenes Messgerät nach einem der vorhergehenden Ansprüche, **gekennzeichnet durch** eine Füllstandausgabeeinheit (40a; 40b; 40c; 40d) ausgebildet zu einer Ausgabe eines Füllstandes der Aufnahmeeinheit (16a; 16b; 16c; 16d).

8. Handgehaltenes Messgerät nach einem der vorhergehenden Ansprüche, **dadurch gekennzeichnet, dass** die Aufnahmeeinheit (16c; 16d) eine, insbesondere einen Aufnahmeraum (38c; 38d) zumindest teilweise begrenzende, zumindest teilweise transparente Wandung (42c; 42d) ausgebildet zur Durchführung einer optischen Messung, insbesondere NIR-Spektroskopie, der Körperflüssigkeit aufweist.

9. Handgehaltenes Messgerät nach einem der vorhergehenden Ansprüche, **dadurch gekennzeichnet, dass** die Aufnahmeeinheit (16c; 16d) zumindest ein beweglich gelagertes Aufnahmeelement (44c, 46c; 44d, 46d) ausgebildet zur Aufnahme der Körperflüssigkeit umfasst.

10. Handgehaltenes Messgerät nach einem der vorhergehenden Ansprüche, **dadurch gekennzeichnet, dass** die Aufnahmeeinheit (16c; 16d) zumindest zwei, insbesondere mit einer maximalen Winkelabweichung von kleiner als 8° senkrecht zu einer Punktionsrichtung (48c; 48d), beweglich gelagerte Aufnahmeelemente (44c, 46c; 44d, 46d) aufweist, welche zur Aufnahme der Körperflüssigkeit, insbesondere innerhalb der Aufnahmeeinheit (16c; 16d), austauschbar sind.

11. Handgehaltenes Messgerät nach einem der vorhergehenden Ansprüche, **dadurch gekennzeichnet, dass** die Punktionseinheit (18d) zumindest zwei, insbesondere mit einer maximalen Winkelabweichung von kleiner als 8°senkrecht zu einer Punktionsrichtung (48d), beweglich gelagerte Punktionselemente (20d, 21d) aufweist, welche insbesondere gemeinsam mit beweglich gelagerten Aufnahmeelementen (44d, 46d) der Aufnahmeeinheit (16d) austauschbar sind.

12. Verfahren zu einem Betrieb eines handgehaltenen Messgeräts zur Analyse von Körperflüssigkeiten, insbesondere Blut, nach einem der vorhergehenden Ansprüche, umfassend zumindest
- einen Verfahrensschritt, zu einer minimal-invasiven Punktion eines Körpers mittels der Punktionseinheit des handgehaltenen Messgeräts;
- einen Verfahrensschritt, in dem eine aus dem Körper aufgrund der Punktion austretende Körperflüssigkeit durch die Aufnahmeeinheit aufgenommen wird, insbesondere unter Nutzung eines Unterdrucks;
- einen Verfahrensschritt zur Erfassung von Messsignalen der in der Aufnahmeeinheit befindlichen Körperflüssigkeit mittels der Kernspinresonanz-Sensoreinheit.

## Claims

1. Hand-held measuring device designed for analysing body fluids, in particular blood, having at least one nuclear magnetic resonance sensor unit (12a; 12b; 12c; 12d), having at least one evaluation unit (14a; 14b; 14c; 14d) designed for evaluating a measurement signal supplied by the nuclear magnetic resonance sensor unit (12a; 12b; 12c; 12d) and having at least one receiving unit (16a; 16b; 16c; 16d), in particular microcapillary unit, designed for receiving the body fluid, **characterized by** at least one puncture unit (18a; 18b; 18c; 18d) designed for minimally invasive puncturing of a body, in particular a capillary bed of a body.

2. Hand-held measuring device according to Claim 1, **characterized in that** the puncture unit (18a; 18d) has at least one puncture element (20a; 20d, 21d), in particular a lancet, which has a maximum outside diameter (24a; 24d) of less than 1 mm at least in a region (22a; 22d) intended to be punctured.

3. Hand-held measuring device according to Claim 1 or 2, **characterized in that** the puncture unit (18a; 18d) has an, in particular ultrasound-driven, mechanical unit (26a; 26d) for puncturing the body.

4. Hand-held measuring device according to any of the preceding claims, **characterized in that** the puncture unit (18b; 18c) has a laser unit (28b; 28c) for puncturing the body.

5. Hand-held measuring device according to any of the preceding claims, **characterized in that** the receiving unit (16a; 16b; 16c; 16d) has a vacuum unit (30a; 30b; 30c; 30d) for generating a vacuum, which is provided for conveying the body fluid.

6. Hand-held measuring device according to Claim 5, **characterized in that** the vacuum unit (30a; 30b; 30c; 30d) has, for generating a vacuum, at least one heating element (32a; 32b; 32c; 32d), which is provided at least for increasing the temperature of a region (34a; 34b; 34c; 34d) at a receiving opening (36a; 36b; 36c; 38d) of a receiving space (38a; 38b; 38c; 38d) of the receiving unit (16a; 16b; 16c; 16d).

7. Hand-held measuring device according to any of the preceding claims, **characterized by** a filling level output unit (40a; 40b; 40c; 40d) designed for outputting a filling level of the receiving unit (16a; 16b; 16c; 16d).

8. Hand-held measuring device according to any of the preceding claims, **characterized in that** the receiving unit (16c; 16d) has an at least partially transparent wall (42c; 42d), in particular at least partially delimiting a receiving space (38c; 38d), designed for carrying out an optical measurement, in particular NIR spectroscopy, of the body fluid.

9. Hand-held measuring device according to any of the preceding claims, **characterized in that** the receiving unit (16c; 16d) comprises at least one movably mounted receiving element (44c, 46c; 44d, 46d) designed for receiving the body fluid.

10. Hand-held measuring device according to any of the preceding claims, **characterized in that** the receiving unit (16c; 16d) has at least two receiving elements (44c, 46c; 44d, 46d), in particular movably mounted with a maximum angular deviation of less than 8° perpendicular to a puncture direction (48c; 48d), which are interchangeable for receiving the body fluid, in particular within the receiving unit (16c; 16d).

11. Hand-held measuring device according to any of the preceding claims, **characterized in that** the puncture unit (18d) has at least two puncture elements (20d, 21d), in particular movably mounted with a maximum angular deviation of less than 8° perpendicular to a puncture direction (48d), which are interchangeable, in particular together with movably mounted receiving elements (44d, 46d) of the receiving unit (16d).

12. Method for operating a hand-held measuring device for analysing body fluids, in particular blood, according to any of the preceding claims, comprising at least
- one method step for minimally invasive puncturing of a body by means of the puncture unit of the hand-held measuring device;
- one method step in which a body fluid escaping from the body due to the puncture is collected by the receiving unit, in particular using a vacuum;
- one method step for detecting measurement signals of the body fluid present in the receiving unit by means of the nuclear magnetic resonance sensor unit.

## Revendications

1. Appareil de mesure portatif conçu pour analyser des fluides corporels, en particulier le sang, comprenant au moins une unité (12a ; 12b ; 12c ; 12d) de détection par résonance magnétique nucléaire, au moins une unité d'évaluation (14a ; 14b ; 14c ; 14d) conçue pour analyser un signal de mesure fourni par l'unité (12a ; 12b ; 12c ; 12d) de détection par résonance magnétique nucléaire et au moins une unité de réception (16a, 16b ; 16c ; 16d), en particulier une unité micro-capillaire, conçue pour recevoir le fluide corporel, **caractérisé par** au moins une unité de ponction (18a ; 18b ; 18c ; 18d) conçue pour une ponction minimalement invasive d'un corps, en particulier d'un lit capillaire d'un corps.

2. Appareil de mesure portatif selon la revendication 1, **caractérisé en ce que** l'unité de ponction (18a ; 18d) comprend au moins un élément de ponction (20a ; 20d, 21d), en particulier une lancette, qui présente, au moins dans une zone (22a ; 22d) prévue pour la ponction, un diamètre extérieur maximal (24a ; 24d) inférieur à 1mm.

3. Appareil de mesure portatif selon la revendication 1 ou la revendication 2, **caractérisé en ce que** l'unité de ponction (18a ; 18d) pour la ponction du corps comprend une unité mécanique (26a ; 26d), en particulier actionnée par ultrasons.

4. Appareil de mesure portatif selon l'une des revendications précédentes, **caractérisé en ce que** l'unité de ponction (18b ; 18c) pour la ponction du corps comprend une unité laser (28b ; 28c).

5. Appareil de mesure portatif selon l'une des revendications précédentes, **caractérisé en ce que** l'unité de réception (16a ; 16b ; 16c ; 16d) comprend une unité à dépression (30a ; 30b ; 30c ; 30d) destinée à générer une dépression qui est prévue pour le transport du fluide corporel.

6. Appareil de mesure portatif selon la revendication 5, **caractérisé en ce que** l'unité à dépression (30a ; 30b ; 30c ; 30d) destinée à générer une dépression comprend au moins un élément chauffant (32a ; 32b ; 32c ; 32d) qui augmente la température d'une zone (34a ; 34b ; 34c ; 34d) au niveau d'une ouverture de réception (36a ; 36b ; 36c ; 38d) d'un espace de réception (38a ; 38b ; 38c ; 38d) de l'unité de réception (16a ; 16b ; 16c ; 16d).

7. Appareil de mesure portatif selon l'une des revendications précédentes, **caractérisé par** une unité (40a ; 40b ; 40c ; 40d) d'émission de niveau de remplissage conçue pour émettre un niveau de remplissage de l'unité de réception (16a ; 16b ; 16c ; 16d).

8. Appareil de mesure portatif selon l'une des revendications précédentes, **caractérisé en ce que** l'unité de réception (16c ; 16d) présente une paroi (42c ; 42d) au moins partiellement transparente, délimitant au moins partiellement un espace de réception (38c ; 38d), conçue pour effectuer une mesure optique, en particulier une spectroscopie NIR (« proche infrarouge »), du fluide corporel.

9. Appareil de mesure portatif selon l'une des revendications précédentes, **caractérisé en ce que** l'unité de réception (16c ; 16d) comprend au moins un élément de réception (44c, 46c ; 44d, 46d) monté de manière mobile et conçu pour recevoir le fluide corporel.

10. Appareil de mesure portatif selon l'une des revendications précédentes, **caractérisé en ce que** l'unité de réception (16c ; 16d) comprend au moins deux éléments de réception (44c, 46c ; 44d, 46d) mobiles, en particulier avec un écart angulaire maximal inférieur à 8° par rapport à une direction de ponction (48c ; 48d), qui sont interchangeables pour recevoir le fluide corporel, en particulier à l'intérieur de l'unité de réception (16c ; 16d).

11. Appareil de mesure portatif selon l'une des revendications précédentes, **caractérisé en ce que** l'unité de ponction (18d) comprend au moins deux éléments de ponction (20d, 21d) montés mobiles, en particulier avec un écart angulaire maximal inférieur à 8° par rapport à une direction de ponction (48d), qui sont aptes à notamment être interchangés conjointement avec des éléments de réception (44d, 46d) montés mobiles de l'unité de réception (16d).

12. Procédé de fonctionnement d'un appareil de mesure portatif pour l'analyse de fluides corporels, notamment le sang, selon l'une des revendications précédentes, comprenant au moins
- une étape de procédé consistant à effectuer une ponction minimalement invasive d'un corps en utilisant l'unité de ponction de l'appareil de mesure portatif ;
- une étape de procédé consistant à recueillir un fluide corporel s'écoulant du corps à la suite de la ponction à l'aide de l'unité de réception, en particulier en utilisant une dépression ;
- une étape de procédé pour la saisie de signaux de mesure du fluide corporel se trouvant dans l'unité de réception au moyen de l'unité de détection par résonance magnétique nucléaire.
